# EUROPÄISCHE PATENTSCHRIFT

(11) **EP 2 832 308 B1**
(45) Veröffentlichungstag und Bekanntmachung des Hinweises auf die Patenterteilung: **16.01.2019**
(21) Anmeldenummer: 14179246.5
(22) Anmeldetag: 31.07.2014
(51) Int. Cl.: A61B 17/70

(54) **Medizinisches Instrument zum Halten und Handhaben eines chirurgischen Befestigungselements und Wirbelsäulenstabilisierungssystem**
Medical instrument for holding and handling a surgical fastening element and spine stabilising system
Instrument médical destiné à maintenir et à manipuler un élément de fixation chirurgical et système de stabilisation de la colonne vertébrale

(30) Priorität: 02.08.2013 DE 102013108362
(43) Veröffentlichungstag der Anmeldung: 04.02.2015
(73) Patentinhaber: Aesculap AG, 78532 Tuttlingen (DE)
(72) Erfinder: Krüger, Sven, 78647 Trossingen (DE)
(74) Vertreter: Hoeger, Stellrecht & Partner Patentanwälte mbB

(56) Entgegenhaltungen:
- US-A1- 2006 200 132
- US-A1- 2009 171 391
- US-A1- 2011 166 606
- US-A1- 2011 263 945

## Beschreibung

Die vorliegende Erfindung betrifft ein medizinisches Instrument zum Halten und Handhaben eines chirurgischen Befestigungselements, welches einen Befestigungsteil und einen relativ zum Befestigungsteil in einer Montagestellung beweglich gelagerten Halteteil für ein Verbindungselement umfasst, wobei das Instrument ein proximales und ein distales Ende aufweist, eine Längsachse definiert sowie einen Zugteil und einen relativ zu diesem in Richtung der Längsachse bewegbaren Druckteil umfasst, welches proximale Ende temporär mit dem Befestigungsteil in der Montagestellung koppelbar ist, ferner umfassend eine Abkoppeleinrichtung zum aktiven Überführen des Instruments von einer Koppelstellung, in welcher der Zugteil und der Halteteil gekoppelt sind, in eine Abkoppelstellung, in welcher der Zugteil und der Halteteil voneinander trennbar sind, wobei das Instrument eine Sicherungseinrichtung (126) zum Sichern des Instruments (16) in der Koppelstellung umfasst, wobei die Sicherungseinrichtung (126) mindestens ein Zugteilsicherungselement (124) und mindestens ein mit diesem zusammenwirkendes Druckteilsicherungselement (158) umfasst, welche in der Abkoppelstellung außer Eingriff stehen und in der Koppelstellung von einer Entsicherungsstellung, in welcher sie außer Eingriff stehen, in eine Sicherungsstellung bringbar sind, in welcher sie in Eingriff stehen, wobei das mindestens eine Zugteilsicherungselement (124) einen Nutenstein (120) umfasst oder an einem Nutenstein (120) angeordnet oder ausgebildet ist und wobei das Druckteilsicherungselement (158) eine Führungsausnehmung (150) umfasst oder an einer Führungsausnehmung (150) angeordnet oder ausgebildet ist.

Ferner betrifft die vorliegende Erfindung ein Wirbelsäulenstabilisierungssystem umfassend mindestens zwei chirurgische Befestigungselemente und mindestens ein Verbindungselement, wobei mindestens eines der mindestens zwei chirurgischen Befestigungselemente einen Befestigungsteil, einen Halteteil mit einer Verbindungselementaufnahme und ein am Halteteil festlegbares Fixierelement zum Festlegen des Verbindungselements in der Verbindungselementaufnahme umfasst.

Medizinische Instrumente und Wirbelsäulenstabilisierungssysteme der eingangs beschriebenen Art sind beispielsweise aus der DE 20 2011 051 211 U1 bekannt. Ebenso wie das aus der US 8,211,110 B1 bekannte medizinische Instrument wird das in der DE 20 2011 051 211 U1 beschriebene Instrument mit seinem proximalen, dem Patienten zugewandten Ende auf den Halteteil des Befestigungselements, der insbesondere in Form eines polyaxial am Befestigungsteil gelagerten Gabelkopfes ausgebildet sein kann, durch Aufschnappen angekoppelt.

Das Aufschnappen des Instruments auf den Halteteil des Befestigungselements ist in der Regel einfach und sicher zu bewältigen. Problematisch ist jedoch häufig das Lösen des Instruments nach endgültiger Positionierung des Befestigungselements in einem Knochenteil eines Patienten, beispielsweise in einem Pedikel desselben.

Aus der US 2011/0263945 A1 sind minimalinvasive Instrumentensets und Vorrichtungen bekannt. Reduzierungswerkzeuge sind in der US 2011/0166606 A1 offenbart. In der US 2009/0171391 A1 sind Systeme und Verfahren zur spinalen Fixierung beschrieben. Instrumente und Verfahren zum Manipulieren sind aus der US 2006/0200132 A1 bekannt.

Es ist daher eine Aufgabe der vorliegenden Erfindung, ein medizinisches Instrument sowie ein Wirbelsäulenstabilisierungssystem der eingangs beschriebenen Art so zu verbessern, dass das Instrument auf einfache Weise wieder vom Halteteil des Befestigungselements gelöst werden kann.

Diese Aufgabe wird bei einem medizinischen Instrument der eingangs beschriebenen Art erfindungsgemäß dadurch gelöst, dass die Führungsausnehmung (150) einen Führungsabschnitt (152) und einen Sicherungsabschnitt (154) umfasst und dass der Nutenstein (120) in der Entsicherungsstellung mit dem Führungsabschnitt (152) und in der Sicherungsstellung mit dem Sicherungsabschnitt (154) zusammenwirkt.

Die erfindungsgemäß vorgeschlagene Weiterbildung ermöglicht es insbesondere, das medizinische Instrument auf einfache und sichere Weise vom Befestigungselement zu trennen. Beispielsweise ist es möglich, die Abkoppeleinrichtung so auszubilden, dass das Instrument von der Koppelstellung in die Abkoppelstellung ohne Ausübung von Kräften auf den Halteteil überführbar ist. So kann insbesondere sichergestellt werden, dass mit dem Instrument beim Trennen oder Abziehen vom Befestigungselement keine Kräfte auf dieses ausgeübt werden, die die Lage und Position des Befestigungselements in einem Knochenteil in unerwünschter und nachteiliger Weise verändern können. Ferner ist es vorteilhaft, dass das Instrument eine Sicherungseinrichtung zum Sichern des Instruments in der Koppelstellung umfasst. Insbesondere ist es mit einer solchen Sicherungseinrichtung möglich, das Instrument praktisch unverlierbar und temporär untrennbar mit dem Befestigungselement zu verbinden. Dies ist insbesondere deshalb vorteilhaft, weil das Instrument beispielsweise auch zum Einsatz für minimalinvasive Eingriffe vorgesehen sein kann und dabei das Ankoppeln und Entkoppeln von einem Befestigungselement auch ohne direkte Sicht auf den Operationssitus für einen Operateur möglich sein sollte. Auf besonders einfache Weise lässt sich die Sicherungseinrichtung konstruktiv ausbilden, da sie mindestens ein Zugteilsicherungselement und mindestens ein mit diesem zusammenwirkendes Druckteilsicherungselement umfasst, welche in der Abkoppelstellung außer Eingriff stehen und in der Koppelstellung von einer Entsicherungsstellung, in welcher sie außer Eingriff stehen, in eine Sicherungsstellung bringbar sind, in welcher sie in Eingriff stehen. Mit anderen Worten kann das Instrument, wenn es die Koppelstellung einnimmt, in dieser gesichert werden, so dass ein unbeabsichtigtes Lösen oder Trennen des Instruments vom Befestigungselement nicht möglich ist. Hierzu wird das Instrument, wenn es die Koppelstellung einnimmt und insbesondere mit einem Befestigungselement gekoppelt ist, vorzugsweise von der Entsicherungsstellung in die Sicherungsstellung überführt. Insbesondere können das mindestens eine Zugteilsicherungselement und das mindestens eine Druckteilsicherungselement so angeordnet und ausgebildet sein, dass sie in der Sicherungsstellung ein Aufspreizen des proximalen Endes des Zugteils verhindern. Damit kann aber auch das Lösen des Instruments vom Befestigungselement verhindert werden. Mit anderen Worten ist das Instrument an das Befestigungselement in der Sicherungsstellung gesichert gekoppelt. Günstig ist es, dass das mindestens eine Zugteilsicherungselement einen Nutenstein umfasst oder an einem Nutenstein angeordnet oder ausgebildet ist und wenn das Druckteilsicherungselement eine Führungsausnehmung umfasst oder an einer Führungsausnehmung angeordnet oder ausgebildet ist. Der Nutenstein kann insbesondere in Form eines Vorsprungs mit seitlichen Nuten ausgebildet sein, in die aufeinander zuweisende Kanten oder Vorsprünge der Führungsausnehmung eingreifen können, und zwar in der Sicherungsstellung. Insbesondere kann so auch bei entsprechender Ausbildung von voneinander weg weisenden Nuten an einem Nutenstein eine beidseitige Sicherung und Führung des Nutensteins in der Führungsausnehmung erreicht werden. Vorteilhaft ist es, dass die Führungsausnehmung einen Führungsabschnitt und einen Sicherungsabschnitt umfasst und dass der Nutenstein in der Entsicherungsstellung mit dem Führungsabschnitt und in der Sicherungsstellung mit dem Sicherungsabschnitt zusammenwirkt. So kann die Führungsausnehmung also insbesondere zwei Abschnitte aufweisen, wobei der Nutenstein im Führungsabschnitt lediglich geführt wird, eine Bewegung des Spreizglieds von der Längsachse weg oder auf diese hin jedoch nicht verhindern kann. Insbesondere in der Sicherungsstellung wirkt dann der Nutenstein mit dem Sicherungsabschnitt so zusammen, dass beispielsweise nur noch eine Bewegung parallel zur Längsachse zwischen dem Nutenstein und dem Sicherungsabschnitt möglich ist, nicht jedoch eine Bewegung des Nutensteins von der Längsachse weg oder auf diese hin.

Auf eine besonders einfache und sichere Weise kann das Instrument vom Befestigungselement getrennt werden, wenn die Abkoppeleinrichtung in Form einer Spreizeinrichtung ausgebildet ist zum Aufspreizen eines proximalen Endes des Zugteils. Beispielsweise kann das Zugteil in proximaler Richtung weisende Zugelemente aufweisen, die zudem eine gewisse Elastizität beziehungsweise Flexibilität aufweisen können, welche eine Verschwenkung der proximalen Enden der Zugelemente von der Längsachse des Instruments weg und wieder auf diese hin ermöglicht. Insbesondere kann die Abkoppeleinrichtung derart ausgebildet sein, dass das proximale Ende des Zugteils das Halteteil in der Abkoppelstellung vollständig freigibt und so ein kraftfreies Lösen des Instruments vom Befestigungselement ermöglicht.

Auf besonders einfache Weise lässt sich das Instrument ausbilden, wenn der Zugteil in Form einer Außenhülse ausgebildet ist und wenn der Druckteil eine in der Außenhülse verschiebbar und/oder verdrehbar gelagerte Innenhülse umfasst. Optional kann das Instrument zum Bewegen des Druckteils relativ zum Zugteil eine Betätigungseinrichtung umfassen, die vorzugsweise am distalen Ende des Instruments angeordnet oder ausgebildet ist. Beispielsweise kann die Betätigungseinrichtung einen Drehknopf umfassen, welcher mit dem Zugteil verschraubbar ist, zum Beispiel durch ein Außengewinde am Drehknopf und ein zu diesem korrespondierendes Innengewinde am distalen Ende des Zugteils. Ferner kann der Drehknopf am Druckteil in axialer Richtung gesichert aber relativ zu diesem rotierbar gelagert sein, so dass insgesamt in Folge einer Verdrehung des Drehknopfs der Druckteil relativ zum Zugteil in Richtung der Längsachse verschiebbar ist. Für eine definierte Verschiebebewegung können insbesondere entsprechende Führungselemente vorgesehen sein, die am Zugteil und/oder am Druckteil angeordnet oder ausgebildet sind und miteinander zusammenwirken.

Gemäß einer bevorzugten Ausführungsform der Erfindung kann vorgesehen sein, dass der Zugteil mindestens ein Zugteilkopplungselement umfasst zum kraft- und/oder formschlüssigen Koppeln mit mindestens einem korrespondierend ausgebildeten Halteteilkopplungselement des Halteteils. Insbesondere kann das Halteteilkopplungselement in Form einer Ausnehmung oder eines Rücksprungs am Halteteils ausgebildet sein, das mindestens eine Zugteilkopplungselement in Form eines korrespondieren Vorsprungs mit einer in distaler Richtung weisenden Rückhaltefläche.

Günstig ist es, wenn das mindestens eine Zugteilkopplungselement in Form eines in Richtung auf die Längsachse des Instruments vorstehenden Zugteilkopplungsvorsprungs ausgebildet ist. Ein Zugteilkopplungsvorsprung kann beispielsweise in eine entsprechende Ausnehmung am Halteteil eingreifen. Außerdem ermöglicht es diese Ausgestaltung des Zugteilkopplungselements, das Halteteil mit dem proximalen Ende des Instruments praktisch von außen zu fassen beziehungsweise zu greifen und nach endgültiger Platzierung im Knochenteil in umgekehrter Weise auch wieder freizugeben.

Vorteilhaft ist es, wenn die Abkoppeleinrichtung mindestens ein Spreizglied umfasst, welches von der Koppelstellung in die Abkoppelstellung bringbar ist und umgekehrt durch eine Relativbewegung des Zugteils und des Druckteils parallel zur Längsachse. Beispielsweise kann das Spreizglied in Form eines der oben beschriebenen Zugelemente ausgebildet sein, die in proximaler Richtung weisend vom Zugteil abstehen.

Besonders einfach und kompakt ausbilden lässt sich das Instrument, wenn das mindestens eine Spreizglied am Zugteil angeordnet oder ausgebildet ist. Alternativ ist es auch möglich, das mindestens eine Spreizglied am Druckteil vorzusehen.

Gemäß einer weiteren bevorzugten Ausführungsform der Erfindung kann vorgesehen sein, dass das mindestens eine Spreizglied von der Koppelstellung, in welcher das mindestens eines Zugteilkopplungselement und das mindestens eine Halteteilkopplungselement in Eingriff stehen, in die Abkoppelstellung bringbar ist, in welcher das mindestens eine Zugteilkopplungselement und das mindestens eine Halteteilkopplungselement außer Eingriff stehen. Auf diese Weise ist es insbesondere möglich, mit zwei derart ausgebildeten Spreizgliedem den Halteteil des Befestigungselements zu umgreifen oder im Wesentlichen zu umgreifen, um so eine temporäre Verbindung zwischen dem Instrument und dem Befestigungselement herzustellen.

Auf besonders kompakte Weise lässt sich das Instrument ausbilden, wenn das mindestens eine Spreizglied das mindestens eine Zugteilkopplungselement trägt oder umfasst. Insbesondere steht das mindestens eine Zugteilkopplungselement vom Spreizglied in Richtung auf die Längsachse hin weisend ab.

Vorteilhaft ist es, wenn das mindestens eine Spreizglied beim Übergang von der Koppelstellung in die Abkoppelstellung von einer Längsachse des Instruments weg bewegbar und beim Übergang von der Abkoppelstellung in die Koppelstellung auf die Längsachse hin bewegbar ist. So kann durch eine einfache Ausschwenk- und Einschwenkbewegung des Spreizglieds das Instrument temporär mit dem Befestigungselement gekoppelt und wieder von diesem abgekoppelt werden.

Ferner ist es günstig, wenn die Spreizeinrichtung mindestens ein mit dem mindestens einen Spreizglied zusammenwirkendes Spreizelement umfasst, welches am Druckteil angeordnet oder ausgebildet ist. Insbesondere dann, wenn das mindestens eine Spreizglied am Zugteil angeordnet oder ausgebildet ist, kann durch das mindestens eine am Druckteil angeordnete oder ausgebildete Spreizelement im Zusammenwirken mit dem mindestens einen Spreizglied das Instrument ohne Einwirkung weiterer äußerer Kräfte von der Koppelstellung in die Abkoppelstellung überführt werden und umgekehrt. So lässt sich das Instrument einfach und sicher kräftefrei vom Befestigungselement lösen.

Die Konstruktion des Instruments wird besonders einfach, wenn das mindestens eine Spreizelement eine in distaler Richtung und von der Längsachse weg geneigte Aufgleitfläche für das mindestens eine Spreizglied aufweist oder umfasst. Insbesondere kann die Aufgleitfläche auch mit einem Vorsprung am Spreizglied zusammenwirken, der an der Aufgleitfläche beim Übergang von der Koppelstellung in die Abkoppelstellung aufgleitet.

Vorteilhaft ist es, wenn das mindestens eine Zugteilsicherungselement in Form eines Zugteilsicherungsvorsprungs oder einer Zugteilsicherungsausnehmung ausgebildet ist und wenn das mindestens eine Druckteilsicherungselement in Form einer zum Zugteilsicherungsvorsprung korrespondierenden Druckteilsicherungsausnehmung oder in Form eines zur Zugteilsicherungsausnehmung korrespondierenden Druckteilsicherungsvorsprungs ausgebildet ist. Insbesondere können die zusammenwirkenden Vorsprünge und Ausnehmungen der Sicherungseinrichtung so angeordnet und ausgebildet sein, dass sie eine Bewegung des Spreizglieds von der Längsachse weg oder auf diese hin in der Sicherungsstellung verhindern. Dies kann insbesondere erreicht werden, wenn die Ausnehmungen und Vorsprünge gegenseitig Anschläge bilden, die quer zur Längsachse, insbesondere in radialer Richtung wirken. Beispielsweise können dies nutförmige, sich parallel zur Längsachse erstreckende Ausnehmungen und in diese parallel zur Längsachse einführbare Vorsprünge sein.

Um eine Bewegung des Zugteils und des Druckteils relativ zueinander einerseits zu begrenzen und andererseits gleichzeitig das Instrument von der Koppelstellung in die Abkoppelstellung zu überführen und umgekehrt, ist es günstig, wenn das mindestens eine Spreizelement den Führungsabschnitt proximalseitig verschließt. Mit anderen Worten ist es so insbesondere möglich, das Instrument dann von der Koppelstellung in die Abkoppelstellung zu überführen, wenn das Druckteil relativ zum Zugteil seine distalste Stellung einnimmt. Diese Relativstellung von Zugteil und Druckteil kann zusätzlich auch durch einen weiteren Anschlag vorgegeben werden, der zum Beispiel eine Bewegung des Druckteils in distaler Richtung relativ zum Zugteil begrenzt.

Ferner kann es günstig sein, wenn der Sicherungsabschnitt mindestens eine Hinterschneidung aufweist, welche mit dem Zugteilsicherungsvorsprung oder der Zugteilsicherungsausnehmung in der Sicherungsstellung in Eingriff steht. Durch die Hinterschneidung kann insbesondere auf einfache Weise eine Bewegung des Zugteils oder eines Teils derselben, beispielsweise des mindestens einen Spreizglieds, in einer Richtung quer zur Längsachse, also von der Längsachse weg oder auf diese hin, unterbunden werden.

Gemäß einer weiteren bevorzugten Ausführungsform der Erfindung kann vorgesehen sein, dass das Instrument von der Sicherungsstellung in eine Verriegelungsstellung bringbar ist und umgekehrt und dass in der Verriegelungsstellung der Druckteil relativ zum Zugteil seine proximalste Stellung einnimmt. Die Verriegelungsstellung kann insbesondere diejenige Stellung sein, in der der Druckteil des Instruments auf eine am Befestigungsteil vorgesehene Klemmeinrichtung einwirkt, um eine Relativbewegung des Halteteils und des Befestigungsteils temporär zu blockieren, wie dies beispielsweise in der DE 20 2011 051 211 U1 beschrieben ist. Damit kann mittels des Instruments eine temporäre Festlegung eines beispielsweise bei einer Polyaxialschraube in der Montagestellung um ein Gelenkzentrum relativ zum Befestigungsteil beweglichen Halteteils erfolgen. In der Verriegelungsstellung ist es also insbesondere möglich, eine Polyaxialität des Befestigungselements quasi zu verriegeln oder, mit anderen Worten, temporär zu blockieren.

Günstig ist es, wenn der Druckteil mindestens ein Druckglied aufweist oder umfasst zum Ausüben einer Druckkraft auf eine Klemmeinrichtung des Befestigungsteils zum temporären Blockieren einer Relativbewegung des Befestigungsteils und des Halteteils relativ zueinander in der Verriegelungsstellung. Beispielsweise kann das mindestens eine Druckglied in Form eines oder mehrerer in proximaler Richtung weisender Vorsprünge ausgebildet sein, die beispielsweise geeignet sind, auf Angriffspunkte oder Eingriffselemente an einem Klemmelement der Klemmeinrichtung des Befestigungselements einzuwirken.

Gemäß einer weiteren bevorzugten Ausführungsform der Erfindung kann vorgesehen sein, dass das Instrument eine Demontagesicherungseinrichtung zum temporären Sichern des Druckteils und des Zugteils relativ zueinander in einer Montagestellung umfasst, in welcher der Zugteil und der Druckteil miteinander unverlierbar gekoppelt sind. Mit anderen Worten kann durch die Demontagesicherungseinrichtung insbesondere verhindert werden, dass der Zugteil und der Druckteil von einer Bedienperson versehentlich intraoperativ voneinander getrennt werden können. Ferner kann die Demontagesicherungseinrichtung günstigerweise so ausgebildet sein, dass das mindestens eine erste Demontagesicherungselement und das mindestens eine zweite Demontagesicherungselement nur mit einem Hilfsmittel, beispielsweise einem speziell dafür ausgebildeten Instrument, außer Eingriff gebracht werden können, um das Instrument zu zerlegen, also insbesondere zu Reinigungs- und Sterilisationszwecken den Zugteil und den Druckteil voneinander zu trennen.

Besonders günstig ist es, wenn die Demontagesicherungseinrichtung mindestens ein erstes Demontagesicherungselement und mindestens ein zweites, mit dem ersten Demontagesicherungselement zusammenwirkendes Demontagesicherungselement umfasst, wenn das mindestens eine erste Demontagesicherungselement am Zugteil angeordnet oder ausgebildet ist, wenn das mindestens eine zweite Demontagesicherungselement am Druckteil angeordnet oder ausgebildet ist, wenn das mindestens eine erste Demontagesicherungselement und das mindestens eine zweite Demontagesicherungselement in der Montagestellung miteinander in Eingriff oder in Kontakt stehen und in einer Demontagestellung, in welcher der Zugteil und der Druckteil voneinander trennbar sind, außer Eingriff stehen. Auf diese Weise ist es insbesondere mit minimalem konstruktiven Aufwand möglich, sicherzustellen, dass während eines operativen Eingriffs der Zugteil und Druckteil nicht in unbeabsichtigter Weise voneinander getrennt werden können. Im Idealfall sind lediglich zwei Elemente zur Ausbildung der Demontagesicherungseinrichtung erforderlich, nämlich ein erstes Demontagesicherungselement am Zugteil und ein zweites am Druckteil.

Vorteilhaft ist es, wenn das mindestens eine erste Demontagesicherungselement und das mindestens eine zweite Demontagesicherungselement in der Montagestellung miteinander nur dann in Kontakt oder in Eingriff stehen, wenn das Instrument die Abkoppelstellung einnimmt. Auf diese Weise kann gleichzeitig auch die Abkoppelstellung definiert werden. Insbesondere ist es so möglich, beispielsweise eine maximale Aufspreizung der Spreizglieder des Zugteils zu begrenzen, um eine Beschädigung des Instruments zu vermeiden.

Besonders einfach wird der Aufbau der Demontagesicherungseinrichtung, wenn das mindestens eine erste oder das mindestens eine zweite Demontagesicherungselement in Form eines in radialer Richtung von der Längsachse weg weisenden Vorsprungs und wenn das jeweils andere Demontagesicherungselement in Form eines Anschlags mit einer in distaler Richtung weisenden Anschlagfläche ausgebildet sind. Durch diese Ausgestaltung ist es insbesondere möglich, in der Demontagestellung eine Bewegung des Zugteils und des Druckteils aufeinander zu zu begrenzen. Mit anderen Worten kann so eine distalste Stellung des Druckteils relativ zum Zugteil definiert werden.

Um das Zusammenführen von Zugteil und Druckteil zur Ausbildung des Instruments zu vereinfachen, ist es günstig, wenn der in radialer Richtung von der Längsachse weg weisende Vorsprung in radialer Richtung bewegbar gehalten oder gelagert ist. Beispielsweise kann er so durch Ein- oder Ausschwenken auf die Längsachse hin oder von dieser weg mit dem Anschlag außer Eingriff oder außer Kontakt gebracht werden, um so eine Trennung des Zugteils und des Druckteils voneinander zu ermöglichen.

Besonders einfach und intuitiv handhabbar wird das Instrument, wenn die Demontagesicherungseinrichtung in Form einer Rast- und/oder Schnappverbindungseinrichtung ausgebildet ist mit in der Montagestellung in Eingriff oder in Kontakt stehenden ersten und zweiten Rast- und/oder Schnappgliedern, die einerseits am Druckteil und andererseits am Zugteil angeordnet oder ausgebildet sind. Eine Rast- und/oder Schnappverbindungseinrichtung ermöglicht es, das Zugteil und das Druckteil zusammenzufügen, insbesondere durch Einschieben des Druckteils in den Zugteil, bis die ersten und zweiten Rast- und/ oder Schnappglieder miteinander in Eingriff oder in Kontakt stehen, so dass eine unbeabsichtigte Trennung des Zugteils und des Druckteils voneinander verhindert werden kann.

Um die Handhabung des Instruments zu verbessern, ist es vorteilhaft, wenn die Demontagesicherungseinrichtung im Bereich eines distalen Endes des Instruments angeordnet oder ausgebildet ist. Auf diese Weise ist es eher unwahrscheinlich, dass die Demontagesicherungseinrichtung mit Körpergewebe in Kontakt kommen kann, welches unter Umständen im Bereich der Demontagesicherungseinrichtung eingeklemmt oder verletzt werden könnte. Daher müssen hier keine besonderen Sicherheitsvorkehrungen getroffen werden, um zu vermeiden, dass beim Einsatz des Instruments Körpergewebe durch die Demontagesicherungseinrichtung geschädigt werden kann.

Die eingangs gestellte Aufgabe wird ferner bei einem Wirbelsäulenstabilisierungssystem der eingangs beschriebenen Art erfindungsgemäß dadurch gelöst, dass es eines der oben beschriebenen medizinischen Instrumente zum Halten und Handhaben mindestens eines der chirurgischen Befestigungselemente umfasst.

Ein Wirbelsäulenstabilisierungssystem mit einem der oben beschriebenen, vorteilhaften medizinischen Instrumente zum Halten und Handhaben mindestens eines der chirurgischen Befestigungselemente weist insbesondere auch die oben im Zusammenhang mit bevorzugten Ausführungsformen der Instrumente beschriebenen Vorteile auf. Es ist mit diesen medizinischen Instrumenten insbesondere möglich, Befestigungselemente des Wirbelsäulenstabilisierungssystems auf einfache und sichere Weise zu implantieren. Der Befestigungsteil kann beispielsweise in Form eines beliebigen Knochenankers ausgebildet sein, insbesondere in Form eines mit einem Außengewinde versehenen Schafts. Der Halteteil kann eine beliebige Form aufweisen, die geeignet ist, stab- oder plattenförmige Verbindungselemente aufzunehmen, um zwei auf diese Weise miteinander gekoppelte Befestigungselemente des Wirbelsäulenstabilisierungssystems in einem vorgegebenen Abstand voneinander zu halten.

Vorteilhaft ist es, wenn der Befestigungsteil und der Halteteil in einer Montagestellung beweglich aneinander gehalten sind. Dies ermöglicht es, den Halteteil relativ zum Befestigungsteil so auszurichten, dass die Verbindungselementaufnahme zum Aufnehmen eines Verbindungselements in optimaler Weise ausgerichtet ist. Mit anderen Worten können so Längsachsen des Befestigungsteils und des Halteteils relativ zueinander gekippt und in gewünschter Weise ausgerichtet werden, um einerseits eine optimale Verankerung des Befestigungsteils an einem Knochenteil eines Patienten zu gewährleisten und andererseits eine optimale Ausrichtung und Anordnung eines Verbindungselements. Selbstverständlich ist es alternativ auch möglich, Befestigungselemente zu nutzen, die einen Befestigungsteil und einen Halteteil aufweisen, die relativ zueinander nicht beweglich sind. Insbesondere können auch vollständig einstückig ausgebildete Befestigungselemente vorgesehen sein, deren Halte- und Befestigungsteile relativ zueinander nicht beweglich sind.

Gemäß einer weiteren bevorzugten Ausführungsform der Erfindung kann vorgesehen sein, dass das mindestens eine Befestigungselement mindestens ein Klemmglied umfasst, welches in der Montagestellung am Halteteil beweglich gehalten ist und in einer Implantationsstellung zwischen dem Verbindungselement und dem Befestigungsteil klemmend gehalten ist. Das Klemmglied kann insbesondere Teil einer Klemmeinrichtung des Befestigungselements sein und dazu dienen, eine Beweglichkeit zwischen dem Halteteil und dem Befestigungsteil teilweise oder vollständig einzuschränken. Insbesondere kann es ein solches Klemmglied ermöglichen, mit einem der oben beschriebenen, vorteilhaften medizinischen Instrumente eine Beweglichkeit des Halteteils und des Befestigungsteils relativ zueinander zu blockieren, beispielsweise indem der Druckteil auf das Klemmglied eine Druckkraft ausübt, so dass das Klemmglied gegen das Befestigungselement gedrückt wird, beispielsweise gegen einen kugelförmigen Kopf desselben, wodurch bei hinreichend hohen Klemmkräften eine Verriegelung des Befestigungselements erfolgt, beispielsweise eine Verriegelung des Halteteils und des Befestigungsteils des in Form einer Polyaxialschraube ausgebildeten Befestigungselements.

Die nachfolgende Beschreibung bevorzugter Ausführungsformen der Erfindung dient im Zusammenhang mit den Zeichnungen der näheren Erläuterung. Es zeigen:
- Figur 1:: eine schematische Gesamtansicht bei der Implantation eines Wirbelsäulenstabilisierungssystems;

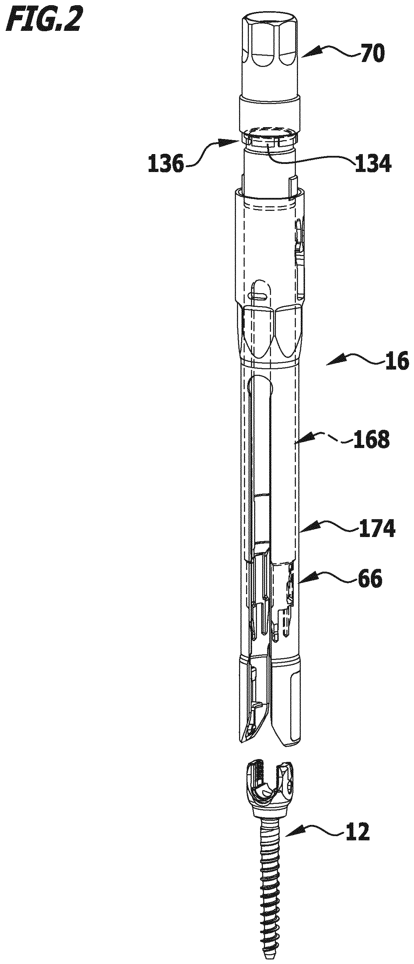

- Figur 2:: eine perspektivische, teilweise durchbrochene Gesamtansicht eines medizinischen Instruments beim Montieren desselben;

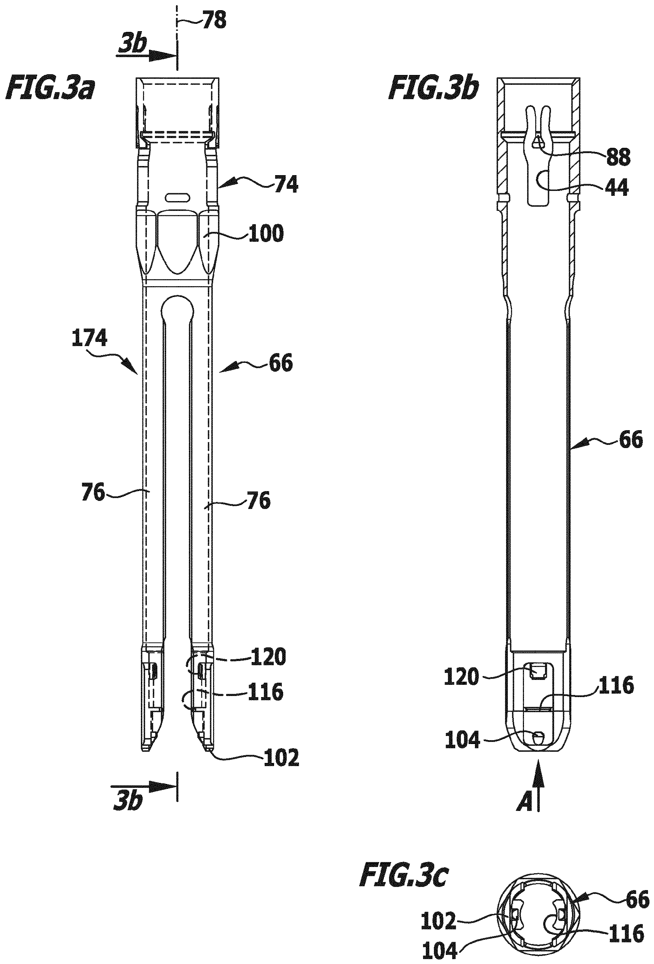

- Figur 3a:: eine Seitenansicht des Zugteils des in Figur 2 dargestellten Instruments;
- Figur 3b:: eine Schnittansicht Längslinie 3b-3b in Figur 3a;
- Figur 3c:: eine Ansicht in Richtung des Pfeils A in Figur 3b;
- Figur 4a:: eine Seitenansicht des Druckteils des in Figur 2 dargestellten Instruments;
- Figur 4b:: eine Seitenansicht des Druckteils in Richtung des Pfeils B in Figur 4a;
- Figur 4c:: eine Ansicht des Druckteils in Richtung des Pfeils C in Figur 4b;

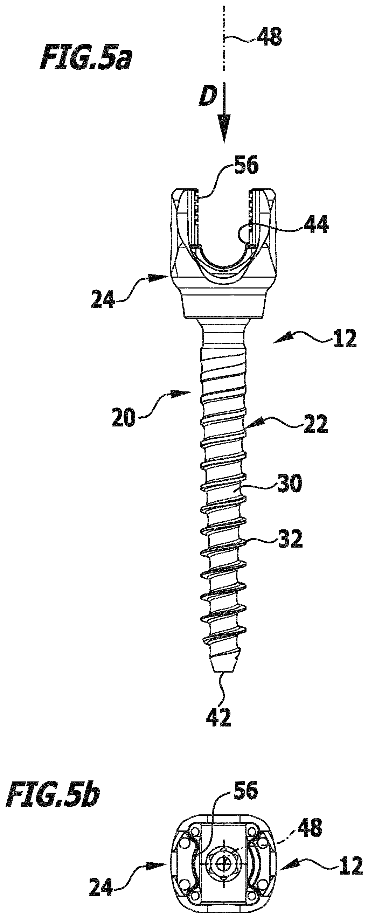

- Figur 5a:: eine Seitenansicht eines Befestigungselements mit Halteteil und relativ zu diesem in der Montagestellung bewegbaren Befestigungsteil;
- Figur 5b:: eine Ansicht in Richtung des Pfeils D in Figur 5a;

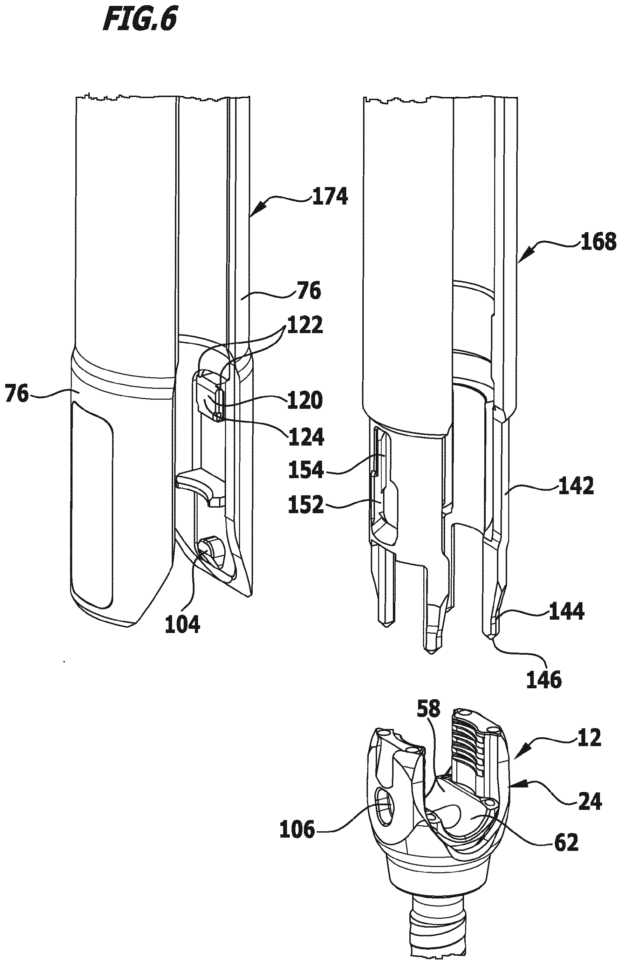

- Figur 6:: eine vergrößerte Ansicht proximaler Enden des Zugteils (links) des Druckteils (rechts) sowie eines distalen Endes des Befestigungselements (unten);

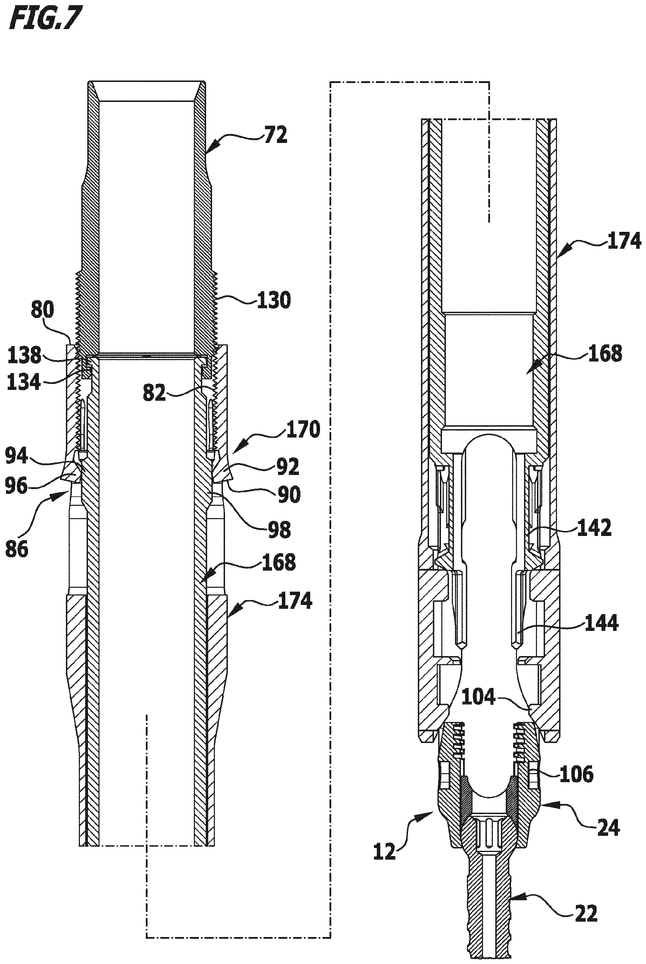

- Figur 7:: eine Längsschnittansicht des Instruments beim Zusammenführen von Druckteil und Zugteil sowie beim Ansetzen des Instruments an ein Befestigungselement;

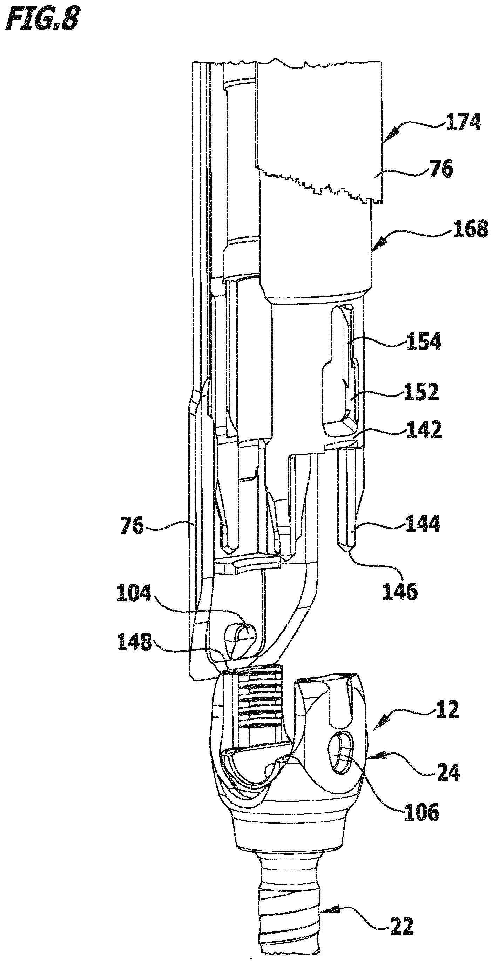

- Figur 8:: eine perspektivische, teilweise durchbrochene Ansicht des rechten Teils der Anordnung in Figur 7;

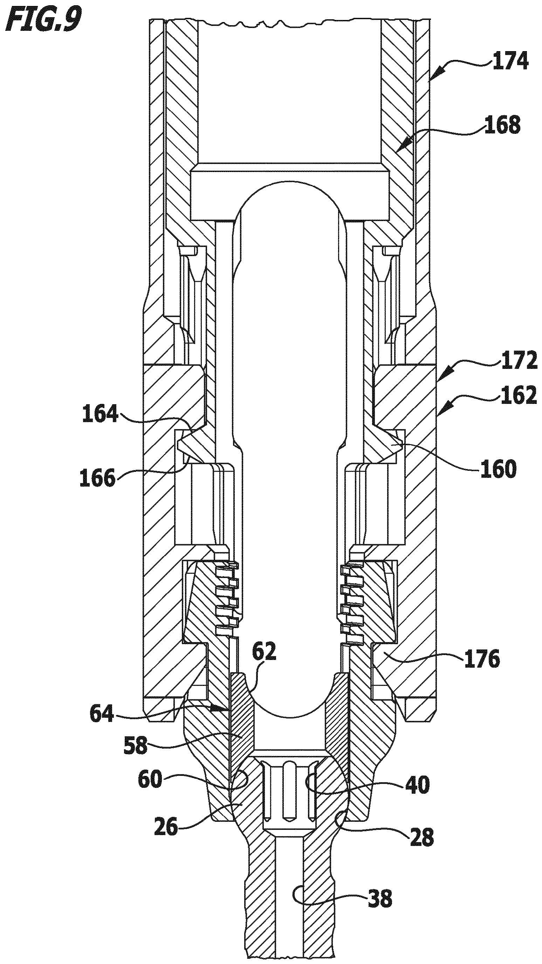

- Figur 9:: eine Schnittansicht des mit dem Befestigungselement gekoppelten Instruments in der Koppelstellung sowie in der Entsicherungsstellung, wobei das Befestigungselement die Montagestellung einnimmt;

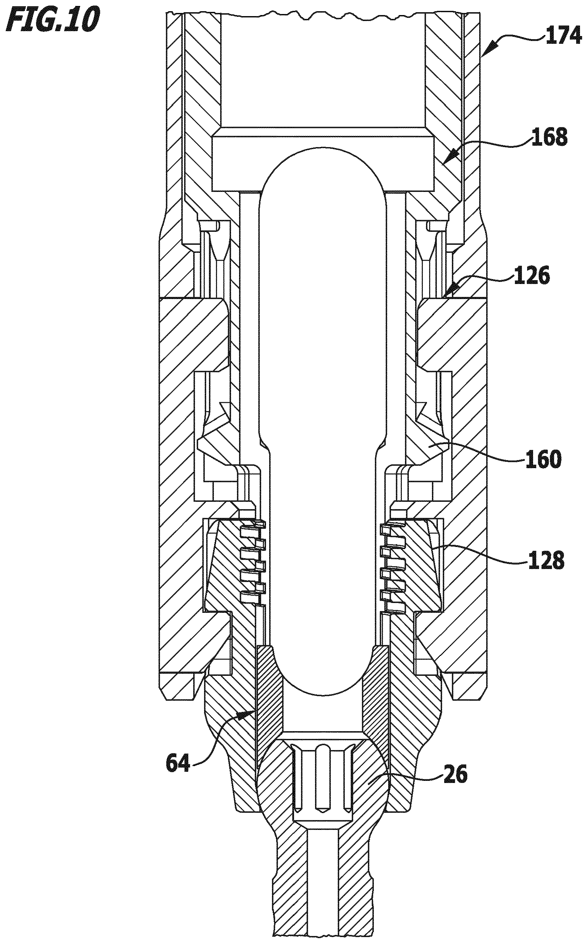

- Figur 10:: eine Ansicht ähnlich Figur 9, wobei das Instrument die Sicherungsstellung einnimmt;

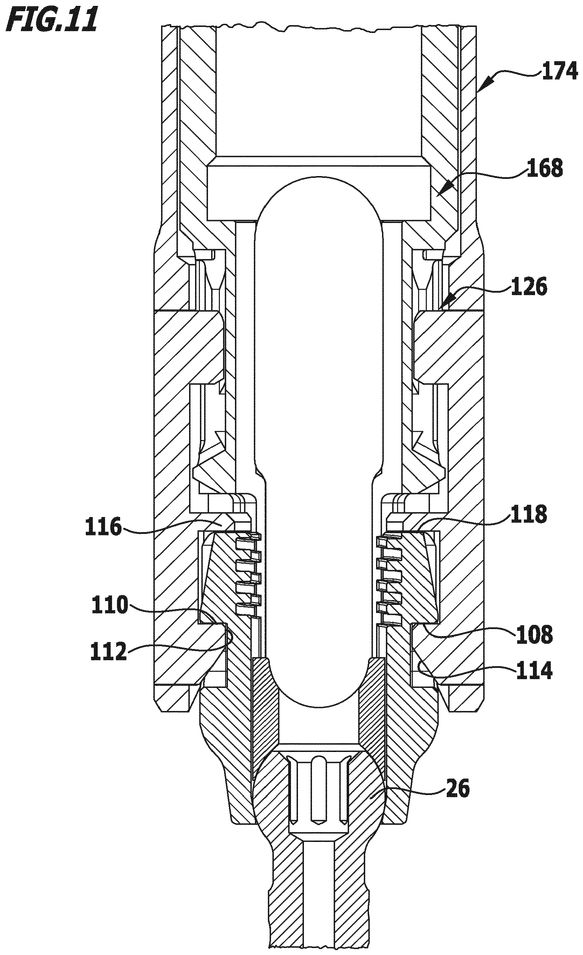

- Figur 11:: eine Ansicht ähnlich Figur 10, wobei das Instrument die Verriegelungsstellung einnimmt, in welcher das Druckteil auf ein Klemmglied des Befestigungselements Druck ausübt;

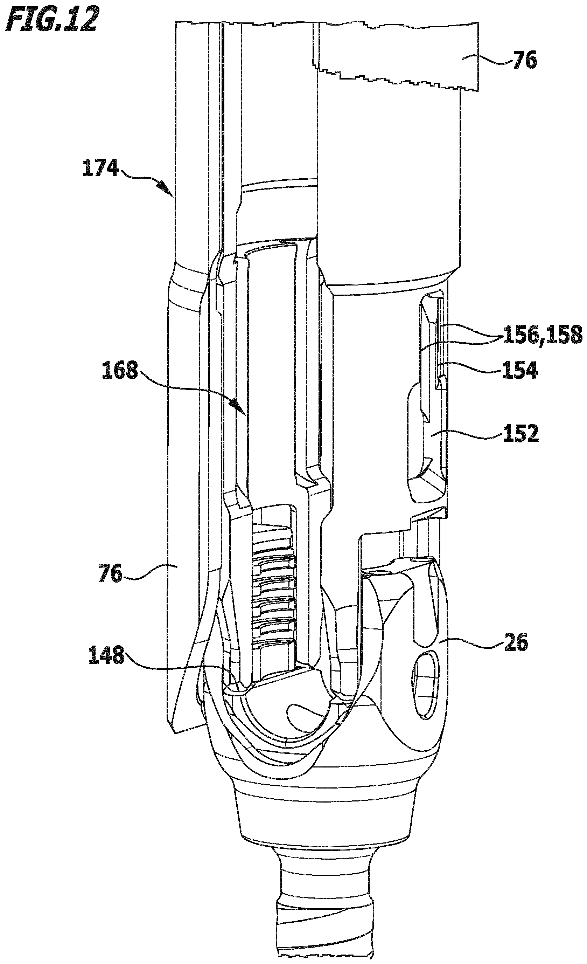

- Figur 12:: eine perspektivische, teilweise durchbrochene Ansicht der Anordnung in Figur 11 in der Verriegelungsstellung.

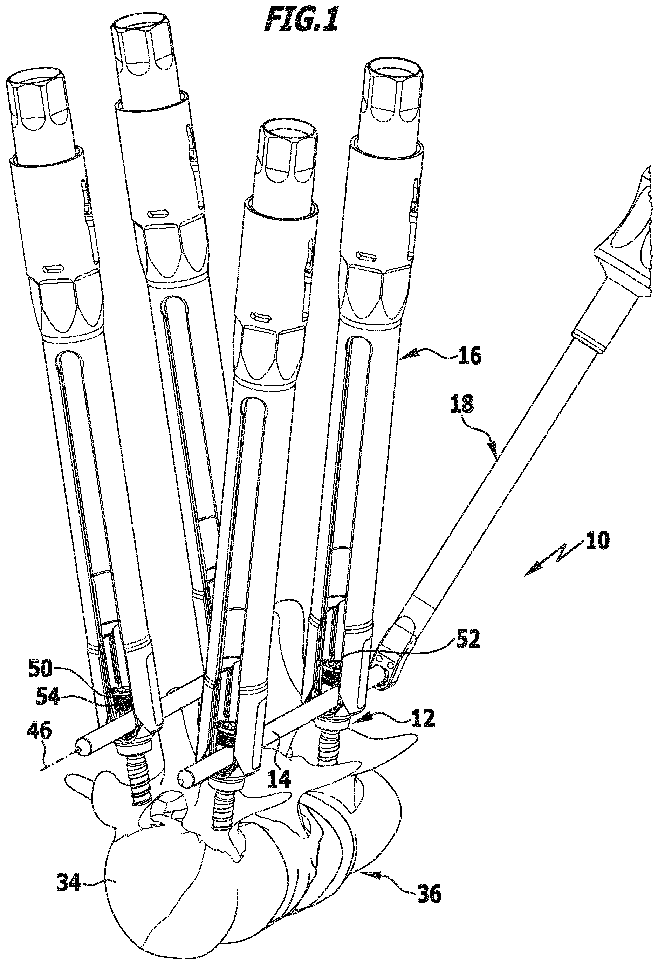

In Figur 1 ist schematisch ein mit dem Bezugszeichen 10 bezeichnetes Wirbelsäulenstabilisierungssystem dargestellt. Es umfasst mindestens zwei chirurgische Befestigungselemente 12, mindestens ein Verbindungselement 14 und mindestens ein medizinisches Instrument 16 zum Halten und Handhaben mindestens eines der Befestigungselemente 12. Optional kann das Wirbelsäulenstabilisierungssystem auch noch ein Halteinstrument 18 zum Halten und Handhaben des Verbindungselements 14 umfassen.

Das Befestigungselement 12 kann insbesondere in Form einer Polyaxialschraube 20 ausgebildet sein, die einen Befestigungsteil 22 und einen Halteteil 24 umfasst. Ein distales Ende des Befestigungsteils 22 ist in Form eines kugeligen Kopfes 26 ausgebildet, welcher in einer korrespondierenden Aufnahme 28 des Halteteils 24 sitzt. Der Befestigungsteil 22 weist einen langgestreckten Schaft 30 auf, welcher mit einem Außengewinde 32 versehen ist, welches vorzugsweise in Form eines selbstschneidenden Knochengewindes ausgebildet ist, um den Befestigungsteil 22 in ein Knochenteil, beispielsweise einen Pedikel eines Wirbels 34 einer Wirbelsäule 36 eines Patienten, einschrauben zu können. Optional kann der Schaft 30 kanuliert sein, das heißt eine Längsbohrung 38 umfassen, die sich von einer Werkzeugaufnahme 40 im Kopf 26 in proximaler Richtung bis zu einem proximalen Ende 42 des Schafts erstreckt.

Der Halteteil 24 ist insgesamt im Wesentlichen hülsenförmig ausgebildet und umfasst eine in distaler Richtung weisend geöffnete Verbindungselementaufnahme 44, in welche ein stab- oder plattenförmiges Verbindungselement 14 mit seiner Längsachse 46 quer zu einer Längsachse 48 des Halteteils 24 eingelegt werden kann. Zum Festlegen des Verbindungselements 14 in der Verbindungselementaufnahme 44 dient ein Fixierelement 50, insbesondere in Form einer Madenschraube, die ein Außengewinde 54 aufweist, welches korrespondierend zu einem Innengewinde 56 am Halteteil 24 ausgebildet ist.

Von distal her kommend ist in das Halteteil 24 ein hülsenförmiges Klemmglied 58 eingesetzt, welches proximalseitig eine hohlkugelige Klemmfläche 60 umfasst, die korrespondierend zum kugeligen Kopf 26 ausgebildet ist und an diesem anliegt. In entgegengesetzter Richtung weisend ist am Klemmglied 58 ferner eine hohlzylindrische Anlagefläche 62 ausgebildet für das vorzugsweise stabförmige Verbindungselement 40, welches einen kreisförmigen oder im Wesentlichen kreisförmigen Querschnitt aufweist. in einer Implantationsstellung, die schematisch in Figur 1 dargestellt ist, ist das Fixierelement 50 in den Halteteil 24 von distal her kommend eingeschraubt und drückt direkt gegen das in die Verbindungselementaufnahme 44 eingesetzte Verbindungselement 14, welches wiederum das Klemmglied 58 einer insgesamt mit dem Bezugszeichen 64 bezeichneten Klemmeinrichtung des Befestigungselements 12 gegen den Befestigungsteil 22, nämlich dessen Kopf 26, drückt, so dass dieser in der Aufnahme 28 verklemmt wird. Auf diese Weise kann die Polyaxialschraube 20 in gewünschter Weise blockiert beziehungsweise verriegelt werden, so dass der Befestigungsteil 22 und der Halteteil 24 mit ihren Längsachsen gegeneinander ausgerichtet und relativ zueinander temporär oder dauerhaft festgelegt werden können.

Zum Halten und Handhaben des Befestigungselements 12 dient das Instrument 16. Es umfasst bei den in den Figuren dargestellten Ausführungsbeispielen drei Teile, nämlich einen in Form einer Außenhülse 174 ausgebildeten Zugteil 66, einen Druckteil 68 sowie ein Betätigungselement 70. Der Zugteil 66, der Druckteil 68 und das Betätigungselement 70 in Form eines Drehknopfs 72 sind alle hülsenförmig beziehungsweise im Wesentlichen hülsenförmig ausgebildet und werden nachfolgend im Einzelnen beschrieben.

Der Zugteil 66 umfasst einen sich über etwa ein Drittel einer Gesamtlänge des Zugteils 66 erstreckenden Hülsenabschnitt 74, von welchem sich in proximaler Richtung zwei armförmige Spreizglieder 76 weg erstrecken. Insgesamt ist das Zugteil 66 symmetrisch zu einer die Längsachse 78 enthaltenden Spiegelebene ausgebildet. Ausgehend vom distalen Ende 80 des Zugteils 66 erstreckt sich ein Innengewindeabschnitt 82 in proximaler Richtung. Zwei sich im Wesentlichen in Längsrichtung erstreckende fensterförmige Durchbrechungen sind bezogen auf die Längsachse 78 einander diametral gegenüberliegend ausgebildet und erstrecken sich teilweise in den Innengewindeabschnitt 82 hinein. Sie bilden einen Teil einer insgesamt mit dem Bezugszeichen 86 bezeichneten Demontagesicherungseinrichtung. Von einem distalen Ende der Durchbrechung steht in proximaler Richtung weisend ein Armglied 88 mit einer in proximaler Richtung weisenden Endfläche 90 ab und bildet einen Anschlag 92 für einen Vorsprung 94 am Druckteil 68. Der Anschlag 92 bildet ein erstes Demontagesicherungselement 96, der Vorsprung 94 ein zweites Demontagesicherungselement 98, deren Funktion nachfolgend noch im Einzelnen beschrieben wird.

Proximalseitig der Durchbrechungen ist am Hülsenabschnitt 74 ein Mehrkantabschnitt 100 ausgebildet mit in radialer Richtung von der Längsachse 78 weg weisenden ebenen Flächenbereichen.

Benachbart an ein proximales Ende 102 des Spreizglieds 76 angrenzend ist ein in Richtung auf die Längsachse 78 vorstehendes Zugteilkopplungselement 104, welches korrespondierend zu einem Halteteilkopplungselement 106 am Halteteil 24 in Form eines Zugteilkopplungsvorsprungs 176 ausgebildet ist. Das Halteteilkopplungselement 106 ist in Form einer von der Längsachse 78 weg weisenden Ausnehmung am Halteteil 24 ausgebildet, die eine in proximaler Richtung weisende Rückhaltefläche 108 für eine in distaler Richtung wiesende Rastfläche 110 des Zugteilkopplungselements 104 aufweist. Proximalseitig ist die in Richtung auf die Längsachse 78 weisende Seitenfläche 112 leicht abgeschrägt und bildet eine Aufgleitfläche 114, die das in Eingriff bringen mit dem Halteteil 24 erleichtert.

Etwas weiter distalseitig des Zugteilkopplungselements 104 steht vom Spreizglied 76 in Richtung auf die Längsachse 78 hin weisend ein Rückhalteglied 116 ab, welches an einem distalen Ende 118 des Halteteils 24 anliegt, wenn das Instrument 16 und das Befestigungselement 12 die Kopplungsstellung einnehmen.

Noch etwas weiter distalseitig als das Rückhalteglied 116 ist am Spreizglied 76 ein in Richtung auf der Längsachse 78 hin weisender Vorsprung in Form eines T-Nutensteins 120 ausgebildet. Dieser ist im Wesentlichen quaderförmig ausgebildet und weist zwei voneinander weg weisende, parallel zur Längsachse verlaufende Nuten 122 auf. Er bildet ein Zugteilsicherungselement 124 einer insgesamt mit dem Bezugszeichen 126 bezeichneten Sicherungseinrichtung 126 zum Sichern des Instruments 16 in der Koppelstellung.

Die Spreizglieder 76 weisen eine hinreichende Elastizität auf, dass proximale Enden derselben beim Aufschieben der Zugteilkopplungselemente 104 auf abgeschrägte Führungsflächen 128 des Halteteils 24 von der Längsachse 78 weg ausschwenken und wieder in Richtung auf diese zurückschnappen können, sobald die Rastfläche 110 die Rückhaltefläche 108 hintergreifen kann.

Das Betätigungselement 70 weist ausgehend von einem proximalen Ende einen Außengewindeabschnitt 130 auf, welcher zum Innengewindeabschnitt 82 korrespondierend ausgebildet ist. Dies ermöglicht es, den Drehknopf 72 von distal herkommend in das Zugteil 66 einzuschrauben. Von einem distalen Ende ausgehend ist auf einer Außenseite des Betätigungselements ein Mehrkant 132 mit von der Längsachse 78 weg weisenden ebenen Flächenbereichen ausgebildet.

In proximaler Richtung weisend stehen vom proximalen Ende des Betätigungselements 70 mehrere über den Umfang verteilte Rastglieder 134 einer Rastverbindung 136 ab, die mit einem Rastglied 138 in Form eines in radialer Richtung von der Längsachse 78 weg weisenden Ringflansches am Druckteil 68 zusammenwirken können. Durch Aufschnappen des Betätigungselements 70 auf ein distales Ende des Druckteils 68 wird eine in axialer Richtung gesicherte Verbindung zwischen dem Betätigungselement 70 und dem Druckteil 68 hergestellt, welche Rastverbindung 136 jedoch eine Verdrehung des Betätigungselements 70 und des Druckteils 68 relativ zueinander ermöglicht.

Etwas weiter proximalseitig des Rastglieds 138 sind auf einer Außenseite des Druckteils 68 die beiden Vorsprünge 94 einander diametral gegenüberliegend bezogen auf die Längsachse 78 ausgebildet. In Umfangsrichtung sind die Vorsprünge 94 so bemessen, dass sie in den Durchbrechungen parallel zur Längsachse 78 geführt werden können.

Nachfolgend wird die Ausgestaltung eines proximalen Endes beziehungsweise eines proximalen Endbereichs des Druckteils 68 näher beschrieben, insbesondere für die Funktion des Instruments 16. Das Druckteil 68 weist ausgehend von seinem proximalen Ende zwei durch Schlitze 140 voneinander getrennte Druckteilabschnitte 142 auf, die jeweils zwei in distaler Richtung weisende, stabförmige Druckglieder 144 tragen. Freie Enden 146 der Druckglieder 144 sind so ausgebildet, dass sie in Vertiefungen 148 am Klemmglied 58 eintauchen können. Die Vertiefungen 148 sind benachbart der Anlagefläche 62 positioniert, so dass das Verbindungselement 14 noch in die Verbindungselementaufnahme 44 eingeführt werden kann, und zwar auch dann, wenn die Druckglieder 144 mit ihren Enden 146 in die Vertiefung in 148 eintauchen.

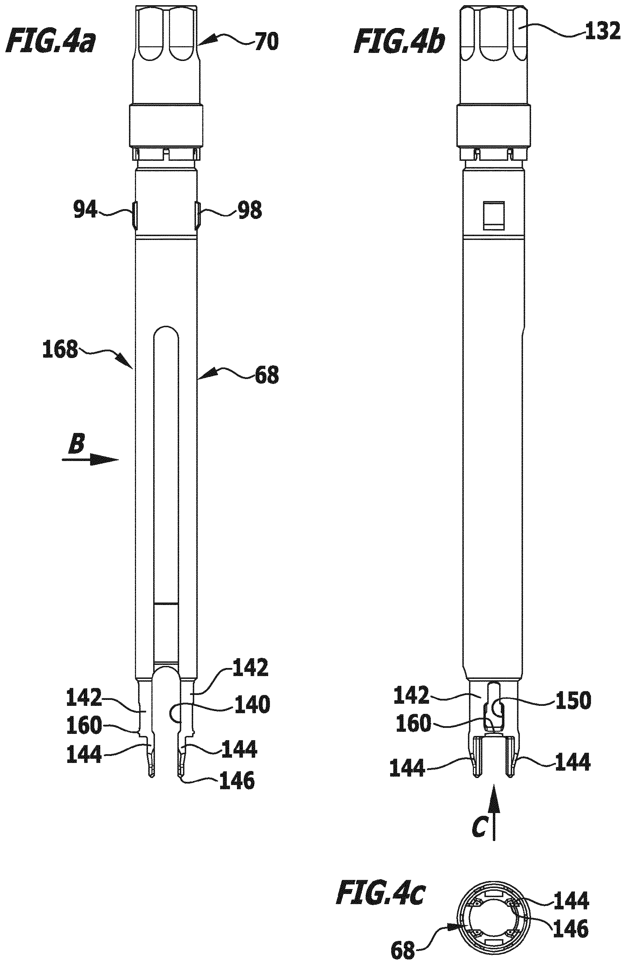

Ferner ist an jedem Druckteilabschnitt 142 eine sich parallel zur Längsachse erstreckende Führungsausnehmung 150 ausgebildet. Diese ist, wie beispielsweise in Figur 4b dargestellt, mit den Vorsprüngen 94 ausgerichtet. Jede Führungsausnehmung 150 weist einen Führungsabschnitt 152 und einen Sicherungsabschnitt 154 auf. Beide sind etwa gleich lang, wobei der Führungsabschnitt 152 etwas breiter ist als der Sicherungsabschnitt 154. Der Sicherungsabschnitt 154 weist zudem eine etwas reduzierte Wandstärke auf. Eine Breite des Sicherungsabschnitts 154 ist so bemessen, dass der T-Nutenstein, welcher frei im Führungsabschnitt 152 in Längsrichtung verschiebbar ist, mit den den Sicherungsabschnitt 154 beidseitig verschmälernden Vorsprüngen 156, welche eine Hinterschneidung am Sicherungsabschnitt 154 begrenzen, zusammenwirken kann, und zwar indem die Vorsprünge 156 in die Nuten 122 eingreifen können. Die Vorsprünge 156 bilden somit Druckteilsicherungselemente 158, die mit den Zugteilsicherungselementen 124 in der Sicherungsstellung des Instruments 16 zusammenwirken.

Ein proximales Ende des Druckteilabschnitts 142 bildet ein die Führungsausnehmung 150 quer zur Längsachse 78 verschließendes Spreizelement 160. Es bildet im Zusammenwirken mit dem Zugteilsicherungselement 124 und den Spreizgliedern 76 eine Abkoppeleinrichtung 162 in Form einer Spreizeinrichtung 172 zum Überführen des Instruments 16 von der Koppelstellung, in welcher der Zugteil 66 und der Halteteil 24 gekoppelt sind, in eine Abkoppelstellung, in welcher der Zugteil 66 und der Halteteil 24 voneinander trennbar sind. Das Spreizelement 160 weist zwei Aufgleitflächen 164 und 166 auf, wobei die Aufgleitfläche 164 im Wesentlichen in distaler Richtung weist, die Aufgleitfläche 166 im Wesentlichen in proximaler Richtung.

Die Montage des Instruments 16 sowie dessen Funktion im Zusammenwirken mit dem Befestigungselement 12 werden nachfolgend beschrieben.

Zur Vorbereitung und Montage des Instruments 16 wird in einem ersten Schritt das Betätigungselement 70 mit dem eine Innenhülse 168 bildenden Druckteil 168 gekoppelt, und zwar durch Aufschnappen des Betätigungselements 70 auf das distale Ende des Druckteils 68.

In einem nächsten Schritt wird nun das Druckteil 68 von distal her kommend mit seinem proximalen Ende voran in das distale Ende des Zugteils 66 eingeschoben, bis der Außengewindeabschnitt 130 und der Innengewindeabschnitt 82 miteinander in Eingriff gelangen. Nun wird durch Drehung des Drehknopfs 72 das Betätigungselement 70 in das Zugteil 66 eingeschraubt, und zwar so weit bis das zweite Demontagesicherungselement 98 in die Durchbrechung einschnappt. Die Durchbrechung ist in ihrer Breite und in Längsrichtung so bemessen, dass in Folge einer Rotation des Drehknopfs 72 das Druckteil 68 relativ zum Zugteil 66 verschoben werden kann, dabei aber axial geführt ist. Eine Bewegung des Druckteils 68 in distaler Richtung relativ zum Zugteil 66 wird begrenzt durch den Anschlag 92, an dem das distale Ende des zweiten Demontagesicherungselements 98 anschlägt. Dies ist der Fall, wenn das Instrument 16 die Abkoppelstellung einnimmt, die noch beschrieben wird. Direkt beim Einschnappen der als Rast- und/oder Schnappverbindungseinrichtung ausgebildeten Demontagesicherungseinrichtung 86 gleitet der T-Nutenstein 120 an der Aufgleitfläche 164 auf, so dass die Spreizglieder 76 von der Längsachse 78 weg nach außen aufgespreizt werden. In dieser Abkoppelstellung ist es möglich, das proximale Ende des Instruments 16 über den Halteteil 24 des Befestigungselements 12 zu schieben, und zwar ohne Kräfte auf das Befestigungselement 12 auszuüben.

Wird der Drehknopf 72 weiter im Uhrzeigersinn gedreht, bewegt sich der Druckteil 68 relativ zum Zugteil 66 weiter in proximaler Richtung, wobei dann die Spreizglieder 76 wieder in Richtung auf die Längsachse 78 zurückfedern können. Ist das proximale Ende des Zugteils 66 über ein Halteteil 24 geschoben, können nun die Zugteilkopplungselemente 104 in die Halteteilkopplungselemente 106 eingreifen, so dass das Instrument 16 temporär mit dem Befestigungselement 12 gekoppelt ist.

Wird der Druckteil 68 relativ zum Zugteil 66 weiter in proximaler Richtung verschoben durch Drehen des Drehknopfs 72 im Uhrzeigersinn, bewegt sich der T-Nutenstein 120 zunächst im Führungsabschnitt 152 der Führungsausnehmung 150. Solange sich der T-Nutenstein 120 vollständig im Bereich des Führungsabschnitts 152 befindet, können die Spreizglieder 76 von der Längsachse 78 weg in radialer Richtung ausschwenken. In dieser Relativstellung, der sogenannten Entsicherungsstellung, von Druckteil 68 und Zugteil 66 zueinander ist es auch möglich, das Instrument 16 auf den Halteteil 24 des Befestigungselements 12 aufzuschieben. Die Zugteilkopplungselemente 104 gleiten bei diesem Ankoppelvorgang an den Führungsflächen 128 des Halteteils 24 auf, bis die Rastfläche 110 die Rückhaltefläche 108 hintergreift und dann das Spreizglied 76 wieder in Richtung auf die Längsachse 78 hin zurückschnappen kann.

Der Druckteil 68 kann noch weiter in proximaler Richtung bezogen auf das Zugteil 66 bewegt werden durch Verdrehen des Drehknopfs 72 im Uhrzeigersinn. Sobald der T-Nutenstein 120 in den Bereich des Sicherungsabschnitts 154 gelangt, greifen die Vorsprünge 156 in die Nuten 122 ein. Die Druckteilsicherungselemente 158 und Zugteilsicherungselemente 124 stehen nun im Eingriff und verhindern eine Bewegung der Spreizglieder 76 von der Längsachse 78 weg. Das Instrument 16 nimmt nun die Sicherungsstellung ein.

Wird der Drehknopf 72 weiter im Uhrzeigersinn gedreht, kann das Instrument 16 schließlich noch in die Verriegelungsstellung überführt werden. In dieser Stellung nimmt der Druckteil 68 bezogen auf den Zugteil 66 seine proximalste Stellung ein. Ist das Instrument 16 mit dem Befestigungselement 12 gekoppelt, tauchen in der Verriegelungsstellung die Enden 146 in die Vertiefungen 148 ein, so dass das Klemmglied 58 der Klemmeinrichtung 64 gegen den Kopf 26 gedrückt wird. Da das Zugteil 66, das mit dem Halteteil 24 gekoppelt ist, in entgegengesetzter Richtung eine Zugkraft auf das Befestigungselement 12 ausübt, wird durch diese Aktivierung der Klemmeinrichtung 64 der Halteteil 24 relativ zum Befestigungsteil 22 verriegelt. Das Befestigungselement 12 ist nun insgesamt starr, der Halteteil 24 ist unbeweglich am Befestigungsteil 22 klemmend gehalten.

Nun kann beispielsweise mit dem Halteinstrument 18 ein Verbindungselement 14 in die Verbindungselementaufnahmen 44 von zwei in benachbarten Wirbeln 34 implantierten Befestigungselementen 12 eingeführt werden, wie dies schematisch in Figur 1 dargestellt ist. Mit nicht dargestellten Instrumenten können die Fixierelemente 50 eingesetzt und mit dem jeweiligen Halteteil 24 verschraubt werden, um das Verbindungselement 14 an den Befestigungselementen 12 endgültig zu fixieren.

Nach Festlegung des Verbindungselements 14 am Befestigungselement 12 kann das Instrument 16 wieder vom Halteteil 24 gelöst werden. Dazu wird der Drehknopf 72 im Gegenuhrzeigersinn verdreht, bis der T-Nutenstein 120 wieder am Spreizelement 160 aufgleitet, wodurch die Spreizglieder 76 in radialer Richtung von der Längsachse 78 weg verschwenkt werden. In dieser Abkoppelstellung liegt der Anschlag 92 mit seiner Endfläche 90 an einer distalen Endfläche des Vorsprungs 94 an. Damit verhindert die Demontagesicherungseinrichtung 86, dass das Druckteil 68 in unbeabsichtigter Weise aus dem Zugteil 66 herausgeschraubt werden kann. Das Lösen der Demontagesicherungseinrichtung 86 ist nur mit einem speziell dafür vorgesehenen Instrument oder Werkzeug möglich. Ein unbeabsichtigtes Trennen des Druckteils 68 vom Zugteil 66 während eines operativen Eingriffs ist somit unmöglich.

Das in der Abkoppelstellung befindliche Instrument 16 kann nun, da das Zugteilkopplungselement 104 das Halteteilkopplungselement 106 freigibt, in distaler Richtung vom Halteteil 24 abgezogen werden.

## Patentansprüche

1. Medizinisches Instrument (16) zum Halten und Handhaben eines chirurgischen Befestigungselements (12), welches einen Befestigungsteil (22) und einen relativ zum Befestigungsteil (22) in einer Montagestellung beweglich gelagerten Halteteil (24) für ein Verbindungselement (14) umfasst, wobei das Instrument (16) ein proximales und ein distales Ende aufweist, eine Längsachse (78) definiert sowie einen Zugteil (66) und einen relativ zu diesem in Richtung der Längsachse (78) bewegbaren Druckteil (68) umfasst, welches proximale Ende temporär mit dem Befestigungselement (12) in der Montagestellung koppelbar ist, ferner umfassend eine Abkoppeleinrichtung (162) zum aktiven Überführen des Instruments (16) von einer Koppelstellung, in welcher der Zugteil (66) und der Halteteil (24) gekoppelt sind, in eine Abkoppelstellung, in welcher der Zugteil (66) und der Halteteil (24) voneinander trennbar sind, wobei das Instrument eine Sicherungseinrichtung (126) zum Sichern des Instruments (16) in der Koppelstellung umfasst, wobei die Sicherungseinrichtung (126) mindestens ein Zugteilsicherungselement (124) und mindestens ein mit diesem zusammenwirkendes Druckteilsicherungselement (158) umfasst, welche in der Abkoppelstellung außer Eingriff stehen und in der Koppelstellung von einer Entsicherungsstellung, in welcher sie außer Eingriff stehen, in eine Sicherungsstellung bringbar sind, in welcher sie in Eingriff stehen, wobei das mindestens eine Zugteilsicherungselement (124) einen Nutenstein (120) umfasst oder an einem Nutenstein (120) angeordnet oder ausgebildet ist und wobei das Druckteilsicherungselement (158) eine Führungsausnehmung (150) umfasst oder an einer Führungsausnehmung (150) angeordnet oder ausgebildet ist, **dadurch gekennzeichnet, dass** die Führungsausnehmung (150) einen Führungsabschnitt (152) und einen Sicherungsabschnitt (154) umfasst und dass der Nutenstein (120) in der Entsicherungsstellung mit dem Führungsabschnitt (152) und in der Sicherungsstellung mit dem Sicherungsabschnitt (154) zusammenwirkt.

2. Medizinisches Instrument nach Anspruch 1, **dadurch gekennzeichnet, dass** die Abkoppeleinrichtung (162) in Form einer Spreizeinrichtung (172) ausgebildet ist zum Aufspreizen eines proximalen Endes des Zugteils (66).

3. Medizinisches Instrument nach Anspruch 1 oder 2, **dadurch gekennzeichnet, dass** der Zugteil (66) in Form einer Außenhülse (174) ausgebildet ist und dass der Druckteil (68) eine in der Außenhülse (174) verschiebbar und/oder verdrehbar gelagerte Innenhülse (168) umfasst.

4. Medizinisches Instrument nach einem der voranstehenden Ansprüche, **dadurch gekennzeichnet, dass** der Zugteil (66) mindestens ein Zugteilkopplungselement (104) umfasst zum kraft- und/oder formschlüssigen Koppeln mit mindestens einem korrespondierend ausgebildeten Halteteilkopplungselement (106) des Halteteils (24).

5. Medizinisches Instrument nach einem der voranstehenden Ansprüche, **dadurch gekennzeichnet, dass** die Abkoppeleinrichtung (162) mindestens ein Spreizglied (76) umfasst, welches von der Koppelstellung in die Abkoppelstellung bringbar ist und umgekehrt durch eine Relativbewegung des Zugteils (66) und des Druckteils (68) parallel zur Längsachse (78).

6. Medizinisches Instrument nach Anspruch 5, **dadurch gekennzeichnet, dass** das mindestens eine Spreizglied (76) am Zugteil (66) angeordnet oder ausgebildet ist.

7. Medizinisches Instrument nach Anspruch 5 oder 6, **dadurch gekennzeichnet, dass** das mindestens eine Spreizglied (76) von der Koppelstellung, in welcher das mindestens eine Zugteilkopplungselement (104) und das mindestens eine Halteteilkopplungselement (106) in Eingriff stehen, in die Abkoppelstellung bringbar ist, in welcher das mindestens eine Zugteilkopplungselement (104) und das mindestens eine Halteteilkopplungselement (106) außer Eingriff stehen.

8. Medizinisches Instrument nach einem der voranstehenden Ansprüche, **dadurch gekennzeichnet, dass** das mindestens eine Zugteilsicherungselement (124) in Form eines Zugteilsicherungsvorsprungs oder einer Zugteilsicherungsausnehmung (122) ausgebildet ist und dass das mindestens eine Druckteilsicherungselement (158) in Form einer zum Zugteilsicherungsvorsprung korrespondierenden Druckteilsicherungsausnehmung oder in Form eines zur Zugteilsicherungsausnehmung (122) korrespondierenden Druckteilsicherungsvorsprungs (156) ausgebildet ist.

9. Medizinisches Instrument nach einem der voranstehenden Ansprüche, **dadurch gekennzeichnet, dass** das mindestens eine Spreizelement (160) den Führungsabschnitt (152) proximalseitig verschließt.

10. Medizinisches Instrument nach einem der voranstehenden Ansprüche, **dadurch gekennzeichnet, dass** der Sicherungsabschnitt (154) mindestens eine Hinterschneidung aufweist, welche mit dem Zugteilsicherungsvorsprung (120) oder der Zugteilsicherungsausnehmung in der Sicherungsstellung in Eingriff steht.

11. Medizinisches Instrument nach einem der voranstehenden Ansprüche, **dadurch gekennzeichnet, dass** das Instrument (16) von der Sicherungsstellung in eine Verriegelungsstellung bringbar ist und umgekehrt und dass in der Verriegelungsstellung der Druckteil (68) relativ zum Zugteil (66) seine proximalste Stellung einnimmt.

12. Medizinisches Instrument nach Anspruch 11, **dadurch gekennzeichnet, dass** der Druckteil (68) mindestens ein Druckglied (144) aufweist oder umfasst zum Ausüben einer Druckkraft auf eine Klemmeinrichtung (64) des Befestigungselements (12) zum temporären Blockieren einer Relativbewegung des Befestigungsteils (22) und des Halteteils (24) relativ zueinander in der Verriegelungsstellung.

13. Medizinisches Instrument nach einem der voranstehenden Ansprüche, **gekennzeichnet durch** eine Demontagesicherungseinrichtung (86) zum temporären Sichern des Druckteils (68) und des Zugteils (66) relativ zueinander in einer Montagestellung, in welcher der Zugteil (66) und der Druckteil (68) miteinander unverlierbar gekoppelt sind.

14. Wirbelsäulenstabilisierungssystem (10) umfassend mindestens zwei chirurgische Befestigungselemente (12) und mindestens ein Verbindungselement (14), wobei mindestens eines der mindestens zwei chirurgischen Befestigungselemente (12) einen Befestigungsteil (22), einen Halteteil (24) mit einer Verbindungselementaufnahme (44) und ein am Halteteil (24) festlegbares Fixierelement (50) zum Festlegen des Verbindungselements (14) in der Verbindungselementaufnahme (44) umfasst, **gekennzeichnet durch** ein medizinisches Instrument (16) zum Halten und Handhaben mindestens eines der chirurgischen Befestigungselemente (12) nach einem der voranstehenden Ansprüche.

15. Wirbelsäulenstabilisierungssystem nach Anspruch 14, **dadurch gekennzeichnet, dass** der Befestigungsteil (22) und der Halteteil (24) in einer Montagestellung beweglich aneinander gehalten sind.

## Claims

1. Medical instrument (16) for holding and handling a surgical securing element (12), which comprises a securing part (22) and a holding part (24), which is assembled such that in an assembled disposition it is movable in relation to the securing part (22), for a connection element (14), wherein the instrument (16) has a proximal and a distal end, defines a longitudinal axis (78) and comprises a pulling part (66) and, movable in relation to the latter in the direction of the longitudinal axis (78), a pushing part (68), wherein the proximal end is temporarily coupleable to the securing element (12) in the assembled disposition, further comprising an uncoupling means (162) for actively transferring the instrument (16) from a coupled position, in which the pulling part (66) and the holding part (24) are coupled, to an uncoupled position, in which the pulling part (66) and the holding part (24) are separable from one another wherein the instrument comprises a securing means (126) for securing the instrument (16) in the coupled position, wherein the securing means (126) comprises at least one pulling part securing element (124) and, cooperating with this, at least one pushing part securing element (158), and in the uncoupled position these are disengaged and in the coupled position they are movable from an unsecured position in which they are disengaged to a secured position in which they are in engagement, wherein the at least one pulling part securing element (124) comprises a grooved block (120) or is arranged or formed on a grooved block (120), and wherein the pushing part securing element (158) comprises a guide recess (150) or is arranged or formed on a guide recess (150), **characterised in that** the guide recess (150) comprises a guide portion (152) and a securing portion (154), and **in that** the grooved block (120) cooperates in the unsecured position with the guide portion (152) and in the secured position with the securing portion (154).

2. Medical instrument according to Claim 1, **characterised in that** the uncoupling means (162) takes the form of a spreading means (172), for spreading a proximal end of the pulling part (66).

3. Medical instrument according to Claim 1 or 2, **characterised in that** the pulling part (66) takes the form of an outer sleeve (174), and **in that** the pushing part (68) comprises an inner sleeve (168) that is assembled displaceably and/or pivotally in the outer sleeve (174).

4. Medical instrument according to one of the preceding claims, **characterised in that** the pulling part (66) comprises at least one pulling part coupling element (104), for force- and/or positively-locking coupling to at least one correspondingly formed holding part coupling element (106) of the holding part (24).

5. Medical instrument according to one of the preceding claims, **characterised in that** the uncoupling means (162) comprises at least one spreading member (76) which is movable from the coupled position to the uncoupled position and vice versa by relative movement of the pulling part (66) and the pushing part (68) parallel to the longitudinal axis (78).

6. Medical instrument according to Claim 5, **characterised in that** the at least one spreading member (76) is arranged or formed on the pulling part (66).

7. Medical instrument according to Claim 5 or 6, **characterised in that** the at least one spreading member (76) is movable from the coupled position, in which the at least one pulling part coupling element (104) and the at least one holding part coupling element (106) are in engagement, to the uncoupled position, in which the at least one pulling part coupling element (104) and the at least one holding part coupling element (106) are disengaged.

8. Medical instrument according to one of the preceding claims, **characterized in that** the at least one pulling part securing element (124) takes the form of a pulling part securing projection or a pulling part securing recess (122), and **in that** the at least one pushing part securing element (158) takes the form of a pushing part securing recess that corresponds to the pulling part securing projection, or a pushing part securing projection (156) that corresponds to the pulling part securing recess (122).

9. Medical instrument according to one of the preceding claims, **characterised in that** the at least one spreading element (160) proximally closes off the guide portion (152).

10. Medical instrument according to one of the preceding claims, **characterised in that** the securing portion (154) has at least one undercut which in the secured position is in engagement with the pulling part securing projection (120) or the pulling part securing recess.

11. Medical instrument according to one of the preceding claims, **characterised in that** the instrument (16) is movable from the secured position into a locked position and vice versa and **in that**, in the locked position, the pushing part (68) adopts its most proximal position in relation to the pulling part (66).

12. Medical instrument according to Claim 11, **characterised in that** the pushing part (68) has or comprises at least one pushing member (144) for exerting a pushing force on a clamping means (64) of the securing element (12), for temporarily blocking a relative movement of the securing part (22) and the holding part (24) in relation to one another in the locked position.

13. Medical instrument according to one of the preceding claims, **characterised by** a dismantling prevention means (86) for temporarily securing the pushing part (68) and the pulling part (66) in relation to one another, in an assembled disposition in which the pulling part (66) and the pushing part (68) are non-detachably coupled to one another.

14. Vertebral column stabilisation system (10) comprising at least two surgical securing elements (12) and at least one connection element (14), wherein at least one of the at least two surgical securing elements (12) comprises a securing part (22), a holding part (24) with a connection element seating (44), and a fixing element (50) which is fixable to the holding part (24), for fixing the connection element (14) in the connection element seating (44), **characterised by** a medical instrument (16) for holding and handling at least one of the surgical securing elements (12) according to one of the preceding claims.

15. Vertebral column stabilisation system according to Claim 14, **characterised in that** the securing part (22) and the holding part (24) are held movable in relation to one another in an assembled disposition.

## Revendications

1. Instrument médical (16) pour le maintien et la manipulation d'un élément de fixation chirurgical (12), qui comprend une partie de fixation (22) et une partie de maintien (24) logée de manière mobile par rapport à la partie de fixation (22) dans une position de montage pour un élément de liaison (14), où l'instrument (16) présente une extrémité proximale et une extrémité distale, définit un axe longitudinal (78) et comprend une partie de traction (66) et une partie de pression (68) mobile par rapport à celle-ci dans la direction de l'axe longitudinal (78), laquelle extrémité proximale peut être couplée temporairement avec l'élément de fixation (12) dans la position de montage, comprenant en outre un dispositif de découplage (162) pour le passage actif de l'instrument (16) d'une position de couplage dans laquelle la partie de traction (66) et la partie de maintien (24) sont couplées, dans une position de découplage dans laquelle la partie de traction (66) et la partie de maintien (24) peuvent être séparées l'une de l'autre, où l'instrument comprend un dispositif de blocage (126) pour le blocage de l'instrument (16) dans la position de couplage, où le dispositif de blocage (126) comprend au moins un élément de blocage de partie de traction (124) et au moins un élément de blocage de partie de pression (158) qui coopère avec celui-ci, qui sont hors de prise dans la position de découplage et peuvent être amenés dans la position de couplage d'une position de déblocage dans laquelle ils sont hors de prise, dans une position de blocage, dans laquelle ils sont en prise, où le au moins un élément de blocage de partie de traction (124) comprend un coulisseau (120) ou est agencé ou formé au niveau d'un coulisseau (120) et où l'élément de blocage de partie de pression (158) comprend un évidement de guidage (150) ou est agencé ou formé au niveau d'un évidement de guidage (150), **caractérisé en ce que** l'évidement de guidage (150) comprend un segment de guidage (152) et un segment de blocage (154) et **en ce que** le coulisseau (120) coopère avec le segment de guidage (152) dans la position de déblocage et avec le segment de blocage (154) dans la position de blocage.

2. Instrument médical selon la revendication 1, **caractérisé en ce que** le dispositif de découplage (162) est formé sous forme d'un dispositif d'expansion (172) pour l'expansion d'une extrémité proximale de la partie de traction (66).

3. Instrument médical selon la revendication 1 ou 2, **caractérisé en ce que** la partie de traction (66) est formée sous forme d'une gaine externe (174) et **en ce que** la partie de pression (68) comprend une gaine interne (168) qui est logée de manière à pouvoir se déplacer et/ou tourner dans la gaine externe (174).

4. Instrument médical selon l'une des revendications précédentes, **caractérisé en ce que** la partie de traction (66) comprend au moins un élément de couplage de partie de traction (104) pour le couplage à assemblage par force et/ou de forme avec au moins un élément de couplage de partie de maintien (106) de la partie de maintien (24) formé de manière correspondante.

5. Instrument médical selon l'une des revendications précédentes, **caractérisé en ce que** le dispositif de découplage (162) comprend au moins un membre d'expansion (76), qui peut être amené de la position de couplage dans la position de découplage et inversement par un mouvement relatif de la partie de traction (66) et de la partie de pression (68) parallèlement à l'axe longitudinal (78).

6. Instrument médical selon la revendication 5, **caractérisé en ce que** le au moins un membre d'expansion (76) est agencé ou formé au niveau de la partie de traction (66).

7. Instrument médical selon la revendication 5 ou 6, **caractérisé en ce que** le au moins un membre d'expansion (76) peut être amené de la position de couplage, dans laquelle le au moins un élément de couplage de partie de traction (104) et le au moins un élément de couplage de partie de maintien (106) sont en prise, dans la position de découplage, dans laquelle le au moins un élément de couplage de partie de traction (104) et le au moins un élément de couplage de partie de maintien (106) sont hors de prise.

8. Instrument médical selon l'une des revendications précédentes, **caractérisé en ce que** le au moins un élément de blocage de partie de traction (124) est formé sous forme d'une protubérance de blocage de partie de traction ou d'un évidement de blocage de partie de traction (122) et **en ce que** le au moins un élément de blocage de partie de pression (158) est formé sous forme d'un évidement de blocage de partie de pression correspondant à la protubérance de blocage de partie de traction ou sous forme d'une protubérance de blocage de partie de pression (156) correspondant à l'évidement de blocage de partie de traction (122).

9. Instrument médical selon l'une des revendications précédentes, **caractérisé en ce que** le au moins un élément d'expansion (160) ferme le segment de guidage (152) du côté proximal.

10. Instrument médical selon l'une des revendications précédentes, **caractérisé en ce que** le segment de blocage (154) présente au moins une contre-dépouille qui est en prise avec la protubérance de blocage de partie de traction (120) ou l'évidement de blocage de partie de traction dans la position de blocage.

11. Instrument médical selon l'une des revendications précédentes, **caractérisé en ce que** l'instrument (16) peut être amené de la position de blocage dans une position de verrouillage et inversement et **en ce que**, dans la position de verrouillage, la partie de pression (68) occupe sa position la plus proximale par rapport à la partie de traction (66).

12. Instrument médical selon la revendication 11, **caractérisé en ce que** la partie de pression (68) présente ou comprend au moins un membre de pression (144) pour exercer une force de pression sur un dispositif de serrage (64) de l'élément de fixation (12) pour le blocage temporaire d'un mouvement relatif de la partie de fixation (22) et de la partie de maintien (24) l'une par rapport à l'autre dans la position de verrouillage.

13. Instrument médical selon l'une des revendications précédentes, **caractérisé par** un dispositif de blocage de démontage (86) pour le blocage temporaire de la partie de pression (68) et de la partie de traction (66) l'une par rapport à l'autre dans une position de montage dans laquelle la partie de traction (66) et la partie de pression (68) sont couplées l'une à l'autre de manière imperdable.

14. Système de stabilisation de la colonne vertébrale (10) comprenant au moins deux éléments de fixation chirurgicaux (12) et au moins un élément de liaison (14), où au moins l'un des au moins deux éléments de fixation chirurgicaux (12) comprend une partie de fixation (22), une partie de maintien (24) avec un logement d'élément de liaison (44) et un élément de fixation (50) qui peut être fixé au niveau de la partie de maintien (24) pour la fixation de l'élément de liaison (14) dans le logement d'élément de liaison (44), **caractérisé par** un instrument médical (16) pour le maintien et la manipulation d'au moins l'un des éléments de fixation chirurgicaux (12) selon l'une des revendications précédentes.

15. Système de stabilisation de la colonne vertébrale selon la revendication 14, **caractérisé en ce que** la partie de fixation (22) et la partie de maintien (24) sont maintenues l'une au niveau de l'autre de manière mobile dans une position de montage.
